(19) **Europäisches Patentamt**
**European Patent Office**
**Office européen des brevets**

(11) **EP 2 465 562 B1**

(12) **EUROPEAN PATENT SPECIFICATION**

(45) Date of publication and mention
of the grant of the patent:
**10.08.2016 Bulletin 2016/32**

(51) Int Cl.:
***A61M 16/00*** *(2006.01)*

(21) Application number: **11170469.8**

(22) Date of filing: **19.06.2011**

(54) **Control of a ventilator machine**

Steuerung einer Ventilatormaschine

Contrôle d'une machine à ventilateur

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.12.2010 DE 102010055243**

(43) Date of publication of application:
**20.06.2012 Bulletin 2012/25**

(73) Proprietor: **Drägerwerk AG & Co. KGaA
23558 Lübeck (DE)**

(72) Inventors:
• **Eger, Marcus
23562 Lubeck (DE)**
• **Hansmann, Hans-Ullrich
23858 Barnitz (DE)**

• **Glaw, Tobias
23558 Lubeck (DE)**
• **Sattler, Frank
23560 Lubeck (DE)**
• **Handzsuj, Thomas
23568 Lübeck (DE)**

(74) Representative: **McCartney, Jonathan William et al
Haseltine Lake LLP
Redcliff Quay
120 Redcliff Street
Bristol BS1 6HU (GB)**

(56) References cited:
**US-A1- 2006 272 641    US-A1- 2009 159 082
US-A1- 2010 083 968    US-A1- 2010 180 898
US-A1- 2010 252 038**

**Description**

The present invention relates to ventilator machine for the automatic control of a ventilator machine.

The aim of the artificial maintaining of respiration with ventilator machines is to relieve the strain on a patient's respiratory muscles and to ensure that there is an adequate supply of oxygen and that carbon dioxide is eliminated to an adequate degree. This can be done by causing the ventilator machine to assume responsibility for the whole of the breathing activity or, in assisting techniques, for part of the breathing activity, existing breathing activity by the patient being assisted or boosted in these latter assisting techniques. For this purpose, the ventilator machines contain a ventilator unit to supply gas for breathing at a pressure which is preset by a control unit. Also present are sensors which sense, as a function of time, pneumatic breathing signals, such for example as the airway pressure, or the flow of the gas for breathing and its volume (which is obtained by integrating the flow), and pass these signals on to the control unit.

In view of the increase in chronic lung disease and the demand for an improved therapy, the non-invasive assistance of breathing with improved interaction between the patient and the ventilator unit is a crucial requirement which needs to be met by modern-day ventilator machines. A significant object to be achieved in this case is the establishing of temporal synchrony between the assistance provided by the machine and the patient's own breathing activity. In the past, what was often done was to sedate patients who were breathing spontaneously to allow the ventilation to be correctly set and to allow synchrony to be forced to exist between the patient and the ventilator machine. From the knowledge which now exists, this procedure is no longer acceptable because risks have to be run of the lungs being damaged by the ventilation.

For improved synchronisation between the patient's breathing activity and the action of the ventilator unit, it is important for the beginning of inspiration and the beginning of expiration to be reliably detected in the patient's breathing activity at an early point in time. Especially in the case of neonates and COPD patients, the detection of phases of breathing is often incorrect or late with conventional methods and results in increased breathing effort even going as far as exhaustion.

For artificial maintaining of respiration which is intended to take account of the patient's breathing activity in an improved way, it is known from DE 10 2007 062 214 B3 not only for pneumatic signals for breathing activity to be sensed but also for electromyographic signals to be picked up by electrodes on the thorax and for electromyographic signals for breathing activity (EMG signals) to be derived from these. These EMG signals are independent of the pneumatic signals for breathing activity and thus constitute an independent source of information which can be used to sense the beginning of inspiration and expiration. However it is not uncommon for the EMG signals to have interference and disruptions, such for example as the ECG signal from the heart, motion artefacts, or what is referred to as crosstalk (muscle activity which has nothing to do with the patient's respiratory system), superimposed on them.

Triggering of breaths on the basis of EMG signals is described in US 6,588,423 B1. In this case the raw EMG signal is pre-processed and, for triggering, a measure of the intensity of the EMG signal (the root mean square) is finally checked, a threshold which is fixed, in relation to one breath, being used.

In practice however even the pre-processed EMG signal is more susceptible to interference or disruption than pneumatic signals (pressure or flow). A susceptibility of this kind to interference or disruption, or a volatility, makes it more difficult to change over between breaths or trigger them when trigger thresholds are used, because the incorrect triggering of too many breaths may occur (what is referred to as auto-triggering), or breaths may be triggered too late (what is referred to as delayed or missed triggering).

Although signals can be prevented from being affected by interference or disruptions by suitable filtering (e.g. by means of the formation of sliding means), for the purpose of making a changeover between phase of ventilation this has the serious disadvantage of causing an additional delay to the signals.

In DE 102 12 497 A1 it is pointed out as a general comment that, at the beginning of an inspiration phase, it is considerably more likely for the inspiration phase to continue than it is at the premature end of the phase and that there is a greater likelihood of a fresh inspiration phase beginning shortly before the end of the expiration phase. The basic rule is said to be that, as it becomes increasingly likely that the event which triggers the phase of ventilation will occur, the threshold for triggering can be lowered, on the one hand because it is becoming less likely that interference or disruptions will have an effect and, what is more, because even if mistriggering happens due to the occurrence of interference the results of this mistriggering will be much less of a nuisance due to the closeness in time to a correct changeover time than a mistriggering at a complete incorrect point in time. However, apart from this no other details are given of how and with what curve over time up to what target point in time a dynamic variation in threshold value over time may be implemented.

US-A-2010/083968 discloses a respiratory support ventilator apparatus that mechanically supports the work of respiration of a patient. The ventilator apparatus is highly portable and optionally wearable so as to promote mobility and physical activity of the patient, and to improve the overall health of the patient. The respiratory support ventilator may monitor a physical activity level and overall health status of the patient, and process this information. The information is used to track efficacy of the ventilation therapy relative to activity level and quality of life, and or to titrate or optimize the ventilation parameters to improve, maintain or optimize the physical activity level and overall health status of the patient.

US-A-2006/272641 discloses a method and a device for detecting the respiratory activity of a person and for controlling the time progression of breathing gas pressure, especially in accordance with physical parameters and considering parameters indicating the momentary physiological condition of the breathing person. The device for detecting the respiratory activity of a person has at least one sensor that provides a first signal indicating the breathing gas flow, wherein at least one signal processing device is provided for processing the first signal. The signal processing device is configured in such a way that said device determines a reference-relation on the basis of the first signal detected during a first time interval. On the basis thereof, the device determines a correlation-relation between the reference-relation and the first signal.; The device generates an output signal indicating the respiratory activity and/or the physiological condition of the breathing person by considering at least the correlation-relation.

US-A-2010/252038 ventilator provides breathing support to a patient in an EMG controlled mode, and has an input that receives an EMG signal representative of breathing activity from the patient and a control unit for controlling the ventilation in dependence of said EMG signal. The ventilator has a registration unit that registers the actual breathing support provided from the ventilator to the patient, and the control unit determines if there is asynchrony between the EMG signal and the breathing activity and, in case of asynchrony, causes a switch from EMG controlled ventilation to a second ventilation mode not dependent on the EMG signal. If synchrony is detected the ventilator can return to EMG controlled ventilation.

US-A-2010/180898 discloses an apparatus for supplying a respiratory gas to a patient within the scope of the diagnosis and/or the treatment of sleep-related breathing disorders includes an electronic signal processing device for generating a pressure control signal on the basis of indicative signals relating to the breathing activity and/or the physiological state of a person. The signal processing device comprises a signal inputting device and an extraction device for generating data field entries according to predefined signal analysis procedures. A pressure signal generator is provided for generating the pressure control signal, taking into account determined data field entries which are at least selected by the extraction device. There is shown in qualitative terms a configuration in respect of time of the respiratory gas flow V, with association of the inspiration phases TI and expiration phases TE recognized on the part of a respiration phase coordinator. There is diagrammatically illustrated a respiratory gas flow curve is the first derivative thereof. In dependence on the entries in a data field and preferably also in dependence on a pressure control signal P predetermined on the part of a pressure presetting device, the criteria for respiration phase recognition are adapted. In the embodiment illustrated, in a first respiration phase classification category K1 respiration phase recognition is affected by threshold value comparison with a threshold value which changes in respect of time with a high level of dynamics from a comparatively large value to a relatively low value. The threshold value is increased again immediately after recognition of a change in respiration phase. Immediate switching back into the preceding respiration phase is avoided by virtue of a dynamically adapted dead time.

The present invention is as claimed in the claims 1.

The present invention provides a ventilator machine which on the one hand makes possible a sensitive changeover to the next phase of ventilation and on the other hand keeps incorrect changeovers from inspiration to expiration or from expiration to inspiration as low as possible, by using a dynamic threshold which is well matched to the ventilation situation at the time.

In accordance with the invention, there is used in an expiration phase, for the changeover to an inspiration phase, a dynamic threshold curve which, after the beginning of the current expiration phase keeps the threshold, for a selected inspiratory refractory period, i.e. until a time $t_{i1}$, at values which are so high that a changeover to inspiration is impossible at such an early time. After this, the threshold curve is lowered in a monotonic decrease to an inspiratory target threshold value at the expected maximum duration $t_{i2}$ of the phase, and a changeover is made to the inspiration phase as soon as the signal for breathing activity rises above the threshold curve for inspiration.

In an inspiration phase, there is used for the changeover to an expiration phase a dynamic threshold curve which, after the beginning of the current inspiration phase, is held for a selected, short expiratory refractory period, i.e. until a time $t_{e1}$, at values which are so low that a changeover to expiration is impossible in this early phase. After this the threshold curve is raised in a monotonic increase to an expiratory target threshold value at the expected maximum duration $t_{e2}$ of the phase, and a changeover is made to the expiration phase as soon as the signal for breathing activity drops between the threshold curve over the expiration.

In this connection, the durations of the inspiration and expiration phases or the duration of a breath (the sum of the inspiration phase and expiration phase for one breath) are each stored. The expected maximum phase durations $t_{i2}$ and $t_{e2}$ can be derived from the distributions of the phase durations (when the reference point is the beginning of the given phase of ventilation) or from the distribution of the breath durations (when the reference point is the beginning of the previous phase of ventilation), preferably in the form of a p quantile of the distribution, the parameter P being fixed in advance and being of a high value close to 1, e.g. 0.95, which means that the time of the expected maximum phase duration is so positioned that in the case of 95% of the previous phases of ventilation the actual end of the phase had already been reached at this time. Alternatively, there may also be assumed to be a Gaussian distribution and the expected maximum phase duration may be fixed as a preset number of standard deviations above the mean in this distribution, e.g. 2.5 $\sigma$.

The times $t_{i1}$ and $t_{e1}$ of the ends of the inspiratory and expiratory refractory periods and the expected maximum phase durations $t_{i2}$ and $t_{e2}$ may be referred to the changeover to the current phase of ventilation as a time zero. Alternatively, the waveform of the signal for breathing activity may be stored for at least the duration of one phase of ventilation and the beginning of the current phase of ventilation may be determined retrospectively by examining the waveform of the signal for breathing activity in a period around the changeover to the current phase of ventilation. As a result of a more exact examination of the waveform of the signal for breathing activity, the actual beginning of the current phase of ventilation can be determined more accurately in retrospect than the triggering time for the changeover of the ventilation machine can in real time. The distributions of the phase durations also give the median values $t_{im}$ and $t_{em}$, in the form of 0.5 quantiles, and these can be considered expected values for the durations of the inspiration and expiration phases.

The target threshold value is determined from the amplitude distributions of the signals for breathing activity at the times of the median phase durations $t_{im}$ and $t_{em}$, $t_{im}$ being the median value of the durations of the inspiratory phases and $t_{em}$ being that of the durations of the expiratory phases. In the amplitude distributions at these times, the target threshold values can be fixed as p quantiles or, if a Gaussian distribution is assumed, as a multiple (not generally a whole-number one) of the standard deviation relative to the mean. The inspiratory target threshold value may for example be fixed as the 0.05 quantile in the amplitude distribution at the time $t_{im}$, i.e. the target threshold value is so positioned that 95% of the amplitudes of the signal for breathing activity are above the target threshold value at the time $t_{im}$. The expiratory target threshold value may be fixed as the 0.95 quantile in the amplitude distribution at the time $t_{em}$, i.e. the target threshold value is so positioned that 95% of the amplitudes of the signal for breathing activity are below the expiratory target threshold value at the time $t_{em}$.

In a preferred embodiment of the invention, the values of the signal for breathing activity are stored at a plurality of times $t_i^j \in [t_{i1}, t_{im}$ (j = 1,...n) during inspiration and a plurality of times $t_e^k \in [t_{e1}, t_{em}]$ (k = 1,...h) during expiration and are stored as amplitude distributions of the values of the signal for breathing activity at this plurality of times. These amplitude distributions can be used as follows to determine the path of the threshold curve to the target threshold value. For this purpose, advantage is first taken of the fact that the distribution of the phase durations constitutes a probability density function, which can be converted (by integration) into a distribution function V(t) which then increases from 0 at the lowest point of the density function (the shortest phase duration observed) to 1 at the extreme end point of the distribution (the longest phase duration observed). The value of this distribution function at any given time says how probable it is that a changeover to the next phase of ventilation has taken place by the time in question. The probability of a changeover, which increases with time, can be converted into thresholds which go down in a corresponding way in the amplitude distributions of the signal for breathing activity, at the plurality of successive times, in such a way that the probability of a changeover given by the distribution function of the phase durations follows the probability with which, according to the amplitude function, the threshold criterion for changeover will have been met.

The thresholds in the amplitude distributions may for example be set in such a way that the distribution function V(t) of the phase durations at the plurality of times $t_i^j \in [t_{i1}, t_{im}]$ and $t_e^k \in [t_{e1}, t_{em}]$ defines a p quantile criterion in the distributions of the signal for breathing activity, where p is a function p = F(V(t)) of V(t). The function which F(V(t)) is of the distribution in this case is one which generally varies substantially linearly with the distribution function, being in the simplest case the identicality F(V(t)) = V(t) for expiration and the reflection F(V(t)) = 1-V(t) for inspiration.

Alternatively, the thresholds may be set as quantities $A(V(t_i^j))$ and $A(V(t_e^k))$ (generally not whole number-quantities) of standard deviations relative to the mean of a Gaussian distribution in such a way that the probability of the phase end given by the distribution of the phase durations is the same as the probability of the threshold criterion being met in the amplitude distributions. A(V(t)) is a function determined in advance which fixes the quantity of standard deviations such that the probability of the phase end given by the distribution of the phase durations is the same as the probability of the threshold criterion being met in the amplitude distributions. This function may for example be so selected that the probability of the phase end given by the distribution function V(t) of the phase durations at a time t follows the probability of the threshold criterion being met in the amplitude distribution, for which purpose it is possible to use the (tabulated) Gaussian error integral

$$\Phi(u) = 1/\left(\sigma\sqrt{2\pi}\right)\int_{-\infty}^{u} e^{\frac{1}{2}\left(\frac{x}{\sigma}\right)^2} dx$$

which states the probability with which there is a value above a value u in a Gaussian distribution, e.g. in a Gaussian distribution, 68% of the entries are within $1\sigma$, 95% are within $2\sigma$ and 99.7% are within $3\sigma$ ($\sigma$ = standard deviation).

As an example, the function F(V(T)) = 1 - V(t) is set in inspiration and the function F(V(T)) = V(t) is set in expiration. In the histograms of the amplitude distributions of the signal for breathing activity at the times $t_i^j \in$

$[t_{i1}, t_{im}]$ and $t_e^k \in [t_{e1}, t_{em}]$, the thresholds are then set in such a way that they fix a $(1-V(t_i^j))$ quantile in the amplitude distribution in inspiration and a $V(t_e^k)$ quantile in expiration. For example, let the first time after $t_i^1$ of the plurality of times be such that the value of the distribution function $V(t_i^1)$ is then 0.05 (corresponding to 0.5%), which is equivalent to a probability of 5% for an actual phase end. At this time, the threshold is then so set in this example in the associated amplitude distribution for the signal for breathing activity that it forms a (1-0.05) quantile, i.e., that 5% of the amplitudes are above the inspiratory threshold which has been set and 95% are below it. At the next time $t_i^2$, let the value of the distribution function of the phase durations then be 0.25. The inspiratory threshold in the amplitude distribution will then be set as a (1-025) or 0.75 quantile at this next time, and 25% of the amplitude values will thus be above the threshold and 75% below it. Hence, the threshold is set in such a way that the probability of a changeover to the next phase of ventilation exactly follows the probability which is found for a phase end from the distribution of the phase durations. This process is continued until such time as the value of the distribution function of the phase durations reaches 0.5, which then corresponds to a threshold in the amplitude distributions at this time the value of whose p quantile is p = 0.5.

The threshold curve may be interpolated in the intervals between the times making up the plurality of times, and for example the threshold curve may in each case be continued linearly to the threshold value at the next time in the plurality of times.

The threshold is then lowered to the target threshold value, which can be derived from the amplitude distribution at the time when the value of the distribution function of the phase durations is 0.5, i.e. even in the period after the distribution function has reached the value of 0.5 the distribution at that time is taken as a basis and the threshold is then lowered to the target threshold value in this distribution. As described above, this target threshold value is fixed as a p quantile in the amplitude distribution at the time $t_{im}$, e.g. as a 0.05 quantile, which means that 95% of the amplitudes of the signal for breathing activity will be above the target threshold value at the time $t_{im}$. The consideration underlying this procedure is that after the mean duration of a phase (corresponding to a value of 0.5 for the distribution function) there are already an increasing number of signals for breathing activity from inspirations which are beginning, and there is thus increasing evidence in the distributions of effects of fresh phases of ventilation which have already recommenced and the distributions thus no longer reflect what happens in phases of ventilation of very long durations which are drawing to a close.

In this case too the continuation to the target values may take place via a plurality of reference points, i.e. at a plurality of times during the interval $[t_{i1}, t_{im}]$ or $[t_{e1}, t_{em}]$, as the case may be, the threshold is continued as the $(1-/V(t))$ quantile in the amplitude distribution for the time $t_{im}$ in the case of the inspiratory threshold and as the $(V(t)$ quantile in the amplitude distribution for the time $t_{em}$ in the case of the expiratory threshold. The threshold curve may once again be interpolated between the times making up the plurality of times.

In this case, too, the following of a path to the target value may take place via a plurality of reference points, i.e. at a plurality of times from the range $[t_{i1}, t_{im}]$ or $[t_{e1}, t_{em}]$, the threshold follows a path defined by the $(1-V(t))$ quantile in the amplitude distribution at the time $t_{im}$ in the case of the expiratory threshold and one defined by the $V(t)$ in the amplitude distribution at the time $t_{em}$ in the case of the expiratory threshold. The threshold curve may again be interpolated in the intervals between the times making up the plurality of times.

The present invention will now be described, by way of example, only, by reference to the accompanying drawings of which:

Fig. 1 is a graph which shows, as a function of time, signals for breathing activity for a plurality of breathing cycles, the signals being superimposed on one another; and

Fig. 2 is a graph which shows the signal for breathing activity as a function of time together with the associated threshold value curves.

In the present example, what is used as a signal for breathing activity is an electromyographic signal (EMG signal) which is picked up from electrodes on the thorax and which represents the muscle activity connected with breathing.

The EMG signal used is preferably a pre-processed one. Pre-processing of this kind of the raw EMG signal is performed in a known manner in such a way that the raw EMG signal is freed of interfering and disrupting signals (e.g. ECG, motion artefacts, mains hum), and finally, envelope curve detection is carried out. Envelope curve detection may for example be performed by "rectification" followed by low pass filtering, the "rectification" being performed by an operation which gives a value (e.g. squaring or pure absolute-value generation). After the low pass filtering, what is then obtained is the envelope curve, i.e. the curve which is an envelope enclosing the waveform of the raw signal. A preferred implementation of the envelope curve detection process is the formation of what is referred to as the RMS (root mean square) over the length of a sliding time slot. The concept of EMG amplitude estimation which can be described by the term "envelope curve detection" is described in detail in Merletti R, Parker P.A.: Electromyography, Physiology, Engineering and Noninvasive Applications, IEEE Press, Wiley Interscience, 2004, Chapter 6.4 et seq, i.e. page 139 ff.

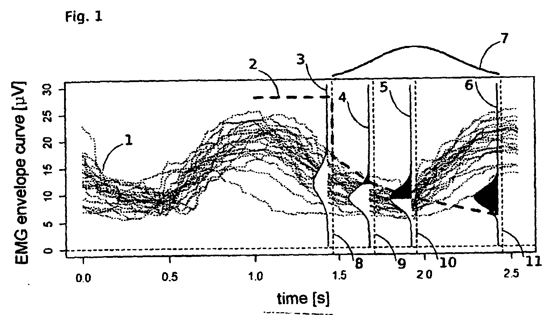

An EMG envelope curve signal of this kind was picked up over a plurality of breathing cycles and, as shown in Fig. 1, was superimposed. When this was done, the individual signals were superimposed in such a way that the exact time of the beginning of inspiration (situated at approx. 1.9 s in Fig. 1) which was found, after the beginning of inspiration, by examining the previous signal for breathing activity was positioned at a common time and, this being the case, the successive phases of ventilation were "synchronised" in the plot in Fig. 1.

In accordance with the invention, there is used in an expiration phase (declining signal for breathing activity) a dynamic threshold curve for the changeover to the next inspiration phase which, after the beginning of the current expiration phase, is kept at high values, and in this example at a high and constant value, for a selected inspiratory refractory period, i.e. until a time $t_{i1}$, to prevent a premature changeover to inspiration. This threshold value curve is identified as 2 in Fig. 1. The inspiratory refractory period is a short period of time which is selected in advance or, as will be explained below, is determined from the signals for breathing activity and which begins at the beginning of the expiration phase and ends at the time $t_{i1}$ and which is so short that it is extremely unlikely that a new inspiration will begin so short a time after the beginning of the expiration. When a value selected in advance is used, the inspiratory refractory period may be 200 ms for example. During this period the threshold 2 is held at a value so high that the possibility of a changeover to inspiration can be virtually ruled out.

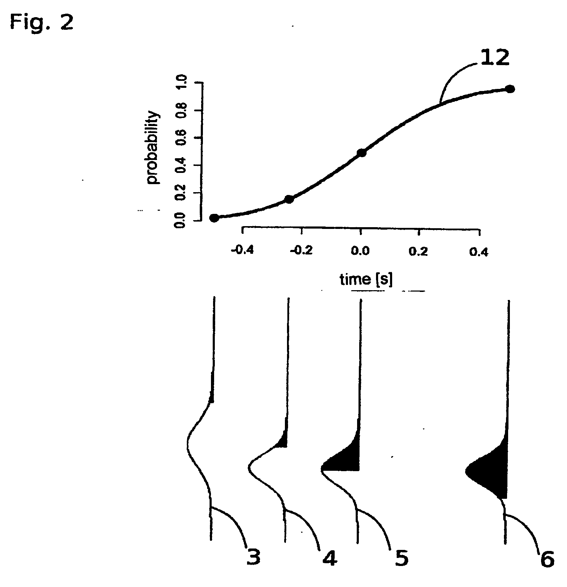

After the inspiratory refractory period, the threshold is lowered in such a way that an optimum threshold value is fixed for the triggering of the next correct inspiration. For this purpose, the distribution of the expiratory phase durations, which is indicated as 7 in Fig. 1, is first considered. This distribution 7 constitutes a probability density function which, when integrated, can also be plotted as a distribution function V(t), as shown at the top of Fig. 2. The distribution function corresponding to the probability density function 7 would then rise, in Fig. 1, from the time identified as 8, from a very small value (which is determined by the p quantile which was used as described above for determining the inspiratory and expiratory refractory times) to a value of close to 1 (which is determined by the p quantile which was used as described above for determining the maximum phase durations) at the time identified as 11, i.e. the probability of the next inspiration beginning rises in a corresponding way over this interval of time. The curve of the distribution function V(t) is identified as 12 at the top of Fig. 2.

In a preferred embodiment, the amplitude distribution of the signal for breathing activity is stored at a plurality of times during the interval between the end $t_{i1}$ of the inspiratory refractory period and the time $t_{im}$ of the mean expected phase duration (the median value of the distribution 7 identified as 10 in Fig. 1); in Fig. 1 these times are, by way of example, three, namely 8, 9 and 10. In the histograms 3, 4 and 5 of the amplitude distributions at these times the threshold is then so set in their respective cases that the threshold for the inspiratory changeover corresponds to a p quantile whose parameter is p = $1-V(t_i^k)$, where $V(t_i^k)$ are the distribution functions of the phase durations at the times k = 8, 9 and 10 as identified in Fig. 1. In more general terms, the p quantiles may be defined as a function of the probability of the distribution function $V(t_i^k)$, i.e. p = $F(V(t_i^k))$.

Alternatively, a Gaussian amplitude distribution may be assumed and the threshold may be defined as $\mu(t_i^k) + k(V(t_i^k)) * \sigma(t_i^k)$, where $\mu(t_i^k)$ is the mean value and $\sigma(t_i^k)$ is the standard deviation of the amplitude distribution at time $t_i^k$. $k(V(t_i^k))$ is a factor which depends on the probability of the distribution function $V(t_i^k)$. If the amplitude distribution is confined to an interval between $\mu$ +/- 2.5 $\sigma$, then k = $-2.5 + (1-V(t_i^k)) * 5$. For very low probability values $V(t_i^k)$, k = 2.5 and the threshold is thus relatively high for $\mu(t_i^k) + 2.5 * \sigma(t_i^k)$. At high probabilities (approaching 1), the result is a low threshold for $\mu(t_i^k) - 2.5 * \sigma(t_i^k)$.

Clearly, what this means is that, after beginning, at time $t_{i1}$, at the topmost edge of the family of curves representing the signals for breathing activity, the threshold then, in the course of the transition to time $t_{im}$, cuts increasingly deeply into the amplitude distributions of the signals for breathing activity in the histograms 3, 4 and 5, as indicated by the parts of the distributions shown in black in Fig. 1 and Fig. 2, which indicate the amplitudes of the signal for breathing activity which exceed the inspiratory threshold. At the time $t_{im}$, the mean duration of the phase, where $V(t_{im}) = 5$, the threshold is so positioned that it forms the median or the 0.5 quantile of the amplitude distribution of the signal for breathing activity at time $t_{im}$. In line with this, the threshold is then situated in the centre of the distribution 5. Once the mean phase duration $t_{im}$ has been reached, there is no point in the threshold continuing to be oriented to histograms of the amplitude distributions at later times, because these later histograms would increasingly contain effects of signals for breathing activity attributable to inspirations which have already begun. The threshold is therefore now lowered to the target threshold value until the expected maximum phase duration $t_{i2}$, identified as 11 in Fig. 1. As was described above in connection with the times $t_i^k$, the target threshold value is determined as a quantile or, if there is assumed to be a Gaussian distribution, as a multiple of the standard deviation relative to the mean, but referred to the amplitude distribution at the time $t_{im}$.

During an inspiration phase, i.e. for the detection of the next expiration, the dynamic expiratory threshold follows a path of the type described above except it is held at low values until the end of the expiratory refractory period $t_{e1}$ and is then raised in a monotonic increase to an expiratory target threshold value, i.e. the threshold follows the reverse path to that shown in Figs. 1 and 2 and moves from below into the distributions until, at the median value $t_{eM}$ of the expiration phase durations it is situated in the centre of the distribution of the amplitudes of the signal for breathing activity, after which it is raised in a monotonic increase to the expiratory target threshold value. In the same way as was described above, this target threshold value is determined as a quantile or, if there is assumed to be a Gaussian distribution, as a multiple of the standard deviation relative to the mean, but referred to the amplitude distribution at the time $t_{em}$.

**Claims**

1. A ventilator machine having:

> a source of gas for breathing;
> a ventilator unit arranged to feed gas for breathing through connecting lines;
> sensors arranged to pick up at least one pneumatic signal for breathing activity and which are connected to a control unit; and in which:
>
>> the control unit is arranged to control the ventilator unit to change over between two alternating phases of ventilation, the two alternating phases of ventilation comprising an inspiration phase and an expiration phase, by, in one phase of ventilation, causing the control unit to examine a sensed respiratory signal for breathing activity for a threshold criterion for the changeover to the next phase of ventilation and changing over from one phase of ventilation to the other when the threshold criterion is met;
>> in the expiration phase, for the changeover to an inspiration phase, a dynamic threshold curve $u_{insp,thresh}(t)$ (2) is kept at sufficiently high values after the beginning of the current expiration phase until the end $t_{i1}$ of a selected inspiratory refractory period to prevent a changeover to inspiration, and thereafter is lowered in a monotonic decrease to an inspiratory target threshold value at an expected time $t_{i2}$ of a maximum duration of the current expiration phase, the control unit being arranged to make the changeover to the inspiration phase when the signal for breathing activity rises above the threshold curve for inspiration; and
>> in the inspiration phase, for the changeover to the expiration phase, a dynamic threshold curve $u_{exp.thresh(t)}$ is held at sufficiently low values after the beginning of the current inspiration phase until the end $t_{e1}$ of a selected expiratory refractory period to prevent a changeover to expiration, and thereafter is raised in a monotonic increase to an expiratory target threshold value at an expected time $t_{e2}$ of a maximum duration of the current inspiration phase, the control unit being arranged to make the changeover to the expiration phase when the signal for breathing activity drops below the threshold curve for expiration; and in which:

> a duration of the inspiration phase and a duration of the expiration phase, or a duration of a breath comprising an inspiration and an expiration phase are each stored, and the expected times $t_{i2}$ and $t_{e2}$ of the maximum phase durations are derived from the distributions of the phase durations using the beginning of the given phase of ventilation as a reference point, or from the distribution of the breath durations when the reference point is the beginning of the previous phase of ventilation.

2. The ventilator machine according to claim 1, in which the expected maximum phase durations $t_{i2}$ and $t_{e2}$ are derived from the distributions of the durations of the inspiration and expiration phases or from the distribution of the breath durations, in the form of a P quantile of these distributions, the parameters $P_{i2}$ and $P_{e2}$ for the quantiles being fixed in advance and the values of these parameters $P_{i2}$ and $P_{e2}$ being at least 0.8.

3. The ventilator machine according to claim 1, in which the expected maximum phase durations $t_{i2}$ and $t_{e2}$ are derived from the distributions of the durations of the inspiration or expiration phases or from the distribution of the breath durations on the assumption that the distributions follow a Gaussian distribution, and the expected maximum phase durations are fixed by a quantity of standard deviations above the mean value which correspond to a preset probability that a maximum phase duration will be situated before the fixed end point of a phase, this probability being at least 0.8.

4. The ventilator machine according to any one of the preceding claims in which the times $t_{i1}$ and $t_{e1}$ of the ends of the inspiratory and expiratory refractory periods and the expected times $t_{i2}$ and $t_{e2}$ of the maximum phase durations are defined relative to the time of the changeover to the current phase of ventilation.

5. The ventilator machine according to any one of claims 1, 2 and 3, in which the waveform of the signal for breathing activity is stored over at least the duration of one half-phase of ventilation, and the beginning of the current phase of ventilation is determined by examining the waveform of the signal for breathing activity in a period around the time of the changeover to the current phase of ventilation, and the times $t_{i1}$ and $t_{e1}$ of the ends of the inspiratory and expiratory refractory periods and the expected times

$t_{i2}$ and $t_{e2}$ of the maximum phase durations are defined relative to the time of the beginning of the current phase of ventilation.

6. The ventilator machine according to any one of the preceding claims, in which the times $t_{i1}$ and/or $t_{e1}$ of the ends of the inspiratory and/or expiratory refractory periods are derived from the distributions of the durations of the inspiration and expiration phases as respective p quantiles of the said distributions, the p quantile values $p_{i1}$ and $p_{e1}$ which are used in this case being selected in advance and their values being less than 0.1.

7. The ventilator machine according to any one of the preceding claims, in which the values of the signal for breathing activity are stored at a plurality of times $t_i^j \in [t_{i1}, t_{im}]$ during inspiration and a plurality of times during expiration $t_e^k \in [t_{e1}, t_{em}]$ (i = 1,...n and k = 1,... m) where $t_{im}$ and $t_{em}$ are the times of the median values of the distributions of the inspiratory and expiratory phase durations, and are stored as amplitude distributions of the values of the signal for breathing activity at this plurality of times, and the inspiratory and expiratory thresholds are so set in these amplitude distributions that the probability of changeover given by the distribution function of the phase durations at the times $t_i^j \in [t_{t1}, t_{im}]$ and $t_e^k \in [t_{e1}, t_{em}]$ follows the probability with which, according to the amplitude distributions at the times $t_i^j$ and $t_e^k$, the threshold criterion for changeover will be met.

8. The ventilator machine according to claim 7, in which the inspiratory threshold $u_{insp,thresh}(t)$ is fixed in such a way that it forms a p quantile where $p = F_1(V(t_i^j))$ in the distribution of the signal for breathing activity at the time $t_i^j$, where $F_1(V(t_i^j))$ is a function of the distribution function of the phase durations which is determined in advance of the current phase of ventilation based on previous phases of ventilation, after which the inspiratory threshold is lowered in such a way that it reaches the inspiratory target threshold value at the expected maximum phase duration $t_{i2}$, and the expiratory threshold $u_{exp.thresh}(t_e^k)$ is fixed in such a way that it forms a p quantile where $p = F_2(V(t_e^k))$ in the distribution of the signal for breathing activity at the time $t_e^k$, where $F_2(V(t_e^k))$ is a function of the distribution function of the phase durations which is determined in advance of the current phase of ventilation based on previous phases of ventilation, after which the expiratory threshold is raised in such a way that it reaches the expiratory target threshold value at the expected maximum phase duration $t_{e2}$.

9. The ventilator machine according to claim 8, in which $F_1(V(t)) = 1-V(t)$ and $F_2(V(t)) = V(t)$ are defined in advance of the current phase of ventilation based on the previous phases of ventilation.

## Patentansprüche

1. Beatmungsgerät, welche Folgendes aufweist:

eine Gasquelle für die Beatmung;
eine Ventilatoreinheit, die zum Zuführen von Gas für die Beatmung durch Verbindungsleitungen geeignet ist;
Sensoren, die zum Erkennen mindestens eines pneumatischen Signals für die Atmungsaktivität geeignet sind und welche mit einer Steuereinheit verbunden sind; und wobei:

die Steuereinheit zur Steuerung der Ventilatoreinheit zum Umschalten zwischen zwei abwechselnden Phasen der Beatmung geeignet ist, wobei die beiden abwechselnden Phasen der Beatmung eine Einatmungsphase und eine Ausatmungsphase umfassen, indem, in einer Phase der Beatmung, die Steuereinheit veranlasst wird, ein erkanntes Atmungssignal für eine Atmungsaktivität auf ein Schwellenkriterium für das Umschalten in die nächste Phase der Beatmung zu prüfen und von einer Phase der Beatmung in die andere umgeschaltet wird, wenn das Schwellenkriterium erfüllt ist;
in der Ausatmungsphase, für die Umschaltung in eine Einatmungsphase, eine dynamische Schwellenkurve $u_{insp,thresh}(t)$ (2) nach dem Beginn der aktuellen Ausatmungsphase bis zum Ende $t_{i1}$ einer ausgewählten Einatmungs-Refraktärzeit auf ausreichend hohen Werten gehalten wird, um eine Umschaltung auf Einatmung zu verhindern, und danach mit einer gleichbleibenden Abnahme auf einen Einatmungszielschwellenwert bei einer angenommenen Zeit $t_{i2}$ einer Maximaldauer der aktuellen Ausatmungsphase gesenkt wird, wobei die Steuereinheit zum Vornehmen der Umschaltung in die Einatmungsphase geeignet ist, wenn das Signal für die Atmungsaktivität über die Schwellenkurve für die Einatmung ansteigt; und
in der Einatmungsphase, für die Umschaltung in die Ausatmungsphase, eine dynamische Schwellenkurve $u_{exp.thresh}(t)$ nach dem Beginn der aktuellen Einatmungsphase bis zum Ende $t_{e1}$ einer ausgewählten Ausatmungs-Refraktärzeit auf ausreichend niedrigen Werten gehalten wird, um eine Umschaltung auf Ausatmung zu verhindern, und danach mit einer gleichbleibenden Zunahme auf einen Ausatmungszielschwellenwert bei einer angenommenen Zeit $t_{e2}$ einer Maximaldauer der aktuellen Einatmungsphase angehoben wird, wobei die Steuereinheit zum Vornehmen der Umschaltung in die Ausatmungsphase geeignet ist, wenn das Signal für die Atmungsaktivität unter die Schwellenkurve für die Ausatmung abfällt; und wobei:

jeweils eine Dauer der Einatmungsphase und eine Dauer der Ausatmungsphase oder eine Dauer eines Atemzugs, der eine Einatmungs- und eine Ausatmungsphase umfasst, gespeichert sind und die angenommenen Zeiten $t_{i2}$ und $t_{e2}$ der maximalen Phasenzeitspannen aus den Verteilungen der Phasenzeitspannen unter Verwendung des Beginns der gegebenen Phase der Beatmung als ein Referenzpunkt oder aus der Verteilung der Atemzugzeitspannen, wenn der Referenzpunkt der Beginn der vorhergehenden Phase der Beatmung ist, abgeleitet werden.

2. Beatmungsgerät nach Anspruch 1, wobei die angenommenen maximalen Phasenzeitspannen $t_{i2}$ und $t_{e2}$ aus den Verteilungen der Zeitspannen der Einatmungs- und Ausatmungsphase oder aus der Verteilung der Atemzugzeitspannen abgeleitet werden, und zwar in Form eines P-Quantils dieser Verteilungen, wobei die Parameter $P_{i2}$ und $P_{e2}$ für die Quantile im Voraus festgelegt werden und die Werte dieser Parameter $P_{i2}$ und $P_{e2}$ mindestens 0,8 betragen.

3. Beatmungsgerät nach Anspruch 1, wobei die angenommenen maximalen Phasenzeitspannen $t_{i2}$ und $t_{e2}$ aus den Verteilungen der Zeitspannen der Einatmungs- oder Ausatmungsphase oder aus der Verteilung der Atemzugzeitspannen unter der Annahme abgeleitet werden, dass die Verteilungen einer Gaußschen Verteilung folgen, und die angenommenen maximalen Phasenzeitspannen durch eine Menge von Standardabweichungen oberhalb des mittleren Wertes, welche einer voreingestellten Wahrscheinlichkeit entsprechen, dass sich eine maximale Phasendauer vor dem festgelegten Endpunkt einer Phase befindet, festgelegt sind, wobei diese Wahrscheinlichkeit mindestens 0,8 beträgt.

4. Beatmungsgerät nach einem der vorhergehenden Ansprüche, wobei die Zeiten $t_{i1}$ und $t_{e1}$ der Enden der Einatmungs- und Ausatmungs-Refraktärzeit und die angenommenen Zeiten $t_{i2}$ und $t_{e2}$ der maximalen Phasenzeitspannen relativ zu der Zeit der Umschaltung in die aktuelle Phase der Beatmung definiert sind.

5. Beatmungsgerät nach einem der Ansprüche 1, 2 und 3, wobei die Wellenform des Signals für die Atmungsaktivität mindestens über die Dauer einer Halbphase der Beatmung gespeichert ist und der Beginn der aktuellen Phase der Beatmung durch eine Prüfung der Wellenform des Signals für die Atmungsaktivität in einem Zeitraum rund um die Zeit der Umschaltung in die aktuelle Phase der Beatmung bestimmt wird und die Zeiten $t_{i1}$ und $t_{e1}$ der Enden der Einatmungs- und Ausatmungs-Refraktärzeit und die angenommenen Zeiten $t_{i2}$ und $t_{e2}$ der maximalen Phasenzeitspannen relativ zu der Zeit des Beginns der aktuellen Phase der Beatmung definiert sind.

6. Beatmungsgerät nach einem der vorhergehenden Ansprüche, wobei die Zeiten $t_{i1}$ und/oder $t_{e1}$ der Enden der Einatmungs- und/oder Ausatmungs-Refraktärzeit aus den Verteilungen der Zeitspannen der Einatmungs- und Ausatmungsphase als entsprechende p-Quantile der Verteilungen abgeleitet werden, wobei die p-Quantil-Werte $p_{i1}$ und $p_{e1}$, welche in diesem Fall verwendet werden, im Voraus ausgewählt werden und wobei ihre Werte weniger als 0,1 betragen.

7. Beatmungsgerät nach einem der vorhergehenden Ansprüche, wobei die Werte des Signals für die Atmungsaktivität zu mehreren Zeiten $t_i^j \in [t_{i1}, t_{im}]$ während der Einatmung und zu mehreren Zeiten während der Ausatmung $t_e^k \in [t_{e1}, t_{em}]$ (i = 1,...n und k = 1,... m) gespeichert werden, wobei $t_{im}$ und $t_{em}$ die Zeiten der Medianwerte der Verteilungen der Einatmungs- und Ausatmungs-Phasenzeitspanne sind und als Amplitudenverteilungen der Werte des Signals für die Atmungsaktivität zu diesen mehreren Zeiten gespeichert werden und die Einatmungs- und Ausatmungsschwelle in diesen Amplitudenverteilungen so eingestellt sind, dass die Wahrscheinlichkeit einer Umschaltung, gegeben durch die Verteilungsfunktion der Phasenzeitspannen zu den Zeiten $t_i^j \in [t_{i1}, t_{im}]$ und $t_e^k \in [t_{e1}, t_{em}]$, der Wahrscheinlichkeit folgt, mit welcher, gemäß der Amplitudenverteilungen zu den Zeiten $t_i^j$ und $t_e^k$, das Schwellenkriterium für eine Umschaltung erfüllt ist.

8. Beatmungsgerät nach Anspruch 7, wobei die Einatmungsschwelle $u_{insp.thresh}(t)$ derart festgelegt ist, dass sie ein p-Quantil bildet, wobei $p = F_1(V(t_i^i))$ in der Verteilung des Signals für die Atmungsaktivität zu der Zeit $t_i^i$, wobei $F_1(V(t_i^i))$ eine Funktion der Verteilungsfunktion der Phasenzeitspannen ist, welche vor der aktuellen Phase der Beatmung auf der Grundlage vorheriger Phasen der Beatmung bestimmt wird, wonach die Einatmungsschwelle derart abgesenkt wird, dass sie den Einatmungszielschwellenwert bei der angenommenen maximalen Phasendauer $t_{i2}$ erreicht, und die Ausatmungs-

schwelle $u_{exp.thresh}(t_e{}^k)$ derart festgelegt ist, dass sie ein p-Quantil bildet, wobei p = $F_2(V(t_e{}^k))$ in der Verteilung des Signals für die Atmungsaktivität zu der Zeit $t_e{}^k$, wobei $F_2(V(t_e{}^k))$ eine Funktion der Verteilungsfunktion der Phasenzeitspannen ist, welche vor der aktuellen Phase der Beatmung auf der Grundlage vorheriger Phasen der Beatmung bestimmt wird, wonach die Ausatmungsschwelle derart angehoben wird, dass sie den Ausatmungszielschwellenwert bei der angenommenen maximalen Phasendauer $t_{e2}$ erreicht.

9. Beatmungsgerät nach Anspruch 8, wobei $F_1(V(t))$ = 1-V(t) und $F_2(V(t))$ = V(t) vor der aktuellen Phase der Beatmung auf der Grundlage vorhergehender Phasen der Beatmung definiert werden.

**Revendications**

1. Respirateur comprenant :

    une source de gaz pour la respiration ;
une unité de ventilation agencée de manière à introduire du gaz pour la respiration par l'intermédiaire de conduites de connexion ;
des capteurs agencés de manière à capter au moins un signal pneumatique de l'activité de respiration et qui sont connectés à l'unité de commande ; et dans lequel :

    l'unité de commande est agencée de façon à commander l'unité de ventilation de manière qu'elle commute successivement entre deux phases alternées de ventilation, les deux phases alternées de ventilation comprenant une phase d'inspiration et une phase d'expiration, en faisant en sorte, dans une première phase de ventilation, que l'unité de commande examine un signal respiratoire détecté de l'activité de respiration par rapport à un critère de seuil pour le passage à la phase suivante de ventilation et en passant d'une phase de ventilation à l'autre quand le critère de seuil est atteint ; dans la phase d'expiration, pour le passage à une phase d'inspiration, une courbe de seuil dynamique $u_{insp,thresh}(t)$ (2) est maintenue à des valeurs suffisamment élevées après le début de la phase d'expiration en cours jusqu'à la fin $t_{i1}$ d'une période réfractaire à l'inspiration sélectionnée afin d'empêcher un passage à une inspiration, et ensuite est abaissée selon une réduction monotone jusqu'à une valeur de seuil cible d'inspiration à un temps prévu $t_{i2}$ d'une durée maximale de la phase d'expiration en cours, l'unité de commande étant agencée pour effectuer le passage à la phase d'inspiration quand le signal de l'activité de respiration augmente au-delà de la courbe de seuil pour l'inspiration ; et

dans la phase d'inspiration, pour le passage à la phase d'expiration, une courbe de seuil dynamique $u_{exp,thresh}(t)$ est maintenue à des valeurs suffisamment faibles après le début de la phase d'inspiration en cours jusqu'à la fin $t_{e1}$ d'une période réfractaire à l'expiration sélectionnée afin d'empêcher un passage à une expiration, et ensuite est relevée selon une augmentation monotone jusqu'à une valeur de seuil cible d'expiration à un temps prévu $t_{e2}$ d'une durée maximale de la phase d'inspiration en cours, l'unité de commande étant agencée pour effectuer le passage à la phase d'expiration quand le signal de l'activité de respiration chute sous la courbe de seuil pour l'inspiration ; et dans lequel :

    chacune parmi la durée de la phase d'inspiration et la durée de la phase d'expiration, ou la durée d'une respiration comprenant une phase d'inspiration et une phase d'expiration, est stockée, et les temps prévus $t_{i2}$ et $t_{e2}$ des durées maximales des phases sont dérivés à partir des distributions des durées des phases par utilisation, en tant que point de référence, du début de la phase de ventilation donnée, ou à partir de la distribution des durées de respiration quand le point de référence est le début de la phase de ventilation précédente.

2. Respirateur selon la revendication 1, dans lequel les durées des phases maximales prévues $t_{i2}$ et $t_{e2}$ sont dérivées à partir des distributions des durées des phases d'inspiration et d'expiration ou à partir de la distribution des durées de respiration, sous la forme d'un quantile P de ces distributions, les paramètres $P_{i2}$ et $P_{e2}$ pour les quantiles étant fixés à l'avance et les valeurs de ces paramètres $P_{i2}$ et $P_{e2}$ étant d'au moins 0,8.

3. Respirateur selon la revendication 1, dans lequel les durées des phases maximales prévues $t_{i2}$ et $t_{e2}$ sont dérivées à partir des distributions des durées des phases d'inspiration et d'expiration ou à partir de la distribution des durées de respiration, avec la supposition que les distributions suivent une distribution gaussienne, et les durées des phases maximales prévues sont fixées par l'ampleur des écarts-types au-dessus de la valeur moyenne qui correspondent à une probabilité préétablie qu'une durée de phase maximale va être située avant le point final fixé d'une phase, cette probabilité étant d'au moins 0,8.

4. Respirateur selon l'une quelconque des revendications précédentes, dans lequel les temps $t_{i1}$ et $t_{e1}$ des fins des périodes réfractaires à l'inspiration et à l'expiration et les temps prévus $t_{e2}$ et $t_{e2}$ des durées des phases maximales sont définis par rapport au moment du passage à la phase actuelle de ventilation.

5. Respirateur selon l'une quelconque des revendications 1, 2 et 3, dans lequel la forme d'onde du signal de l'activité de respiration est stockée sur au moins la durée d'une demi-phase de ventilation, et le début de la phase actuelle de ventilation est déterminé par examen de la forme d'onde du signal de l'activité de respiration dans une période aux alentours du moment du passage à la phase actuelle de ventilation, et les temps $t_{i1}$ et $t_{e1}$ des fins des périodes réfractaires à l'inspiration et à l'expiration et les temps prévus $t_{i2}$ et $t_{e2}$ des durées des phases maximales sont définis par rapport au moment du début de la phase actuelle de ventilation.

6. Respirateur selon l'une quelconque des revendications précédentes, dans lequel les temps $t_{i1}$ et/ou $t_{e1}$ des fins des périodes réfractaires à l'inspiration et/ou à l'expiration sont dérivés à partir des distributions des durées des phases d'inspiration et d'expiration sous la forme de quantiles p respectifs desdites distributions, les valeurs des quantiles p $p_{i1}$ et $p_{e1}$ qui sont utilisées dans ce cas étant choisies à l'avance et leurs valeurs étant inférieures à 0,1.

7. Respirateur selon l'une quelconque des revendications précédentes, dans lequel les valeurs du signal de l'activité de respiration sont stockées plusieurs fois $t_i^j \in [t_{i1}, t_{im}]$ durant l'inspiration et plusieurs fois durant l'expiration $t_e^k \in [t_{e1}, t_{em}]$ (i = 1, ... n et k = 1, ... m), où $t_{im}$ et $t_{em}$ sont les temps des valeurs médianes des distributions des durées des phases d'inspiration et d'expiration, et sont stockées sous la forme de distributions d'amplitude des valeurs du signal de l'activité de respiration pour cette pluralité de temps, et les seuils d'inspiration et d'expiration sont établis dans ces distributions d'amplitude de façon que la probabilité d'un passage, donnée par la fonction de la distribution des durées des phases aux temps $t_i^j \in [t_{i1}, t_{im}]$ et $t_e^k \in [t_{e1}, t_{em}]$, suive la probabilité selon laquelle, en fonction des distributions d'amplitude aux temps $t_i^j$ et $t_e^k$, le critère de seuil pour un passage va être satisfait.

8. Respirateur selon la revendication 7, dans lequel le seuil d'inspiration $u_{insp,thresh}(t)$ est fixé de façon qu'il forme un quantile p où $p = F_1(V(t_i^j))$ dans la distribution du signal de l'activité de respiration au temps $t_i^j$, où $F_1(V(t_i^j))$ est une fonction de la fonction de distribution des durées des phases qui est déterminée à l'avance de la phase actuelle de ventilation sur la base de phases antérieures de ventilation, après quoi le seuil d'inspiration est abaissé de façon qu'il atteigne la valeur de seuil cible d'inspiration à la durée de phase maximale prévue $t_{i2}$, et le seuil d'expiration $u_{exp,thresh}(t_e^k)$ est fixé de façon qu'il forme un quantile p où $p = F_2(V(t_e^k))$ dans la distribution du signal de l'activité de respiration au temps $t_e^k$, où $F_2(V(t_e^k))$ est une fonction de la fonction de distribution des durées des phases qui est déterminée à l'avance de la phase actuelle de ventilation sur la base de phases antérieures de ventilation, après quoi le seuil d'expiration est relevé de façon qu'il atteigne la valeur de seuil cible d'expiration à la durée de phase maximale prévue $t_{e2}$.

9. Respirateur selon la revendication 8, dans lequel $F_1(V(t)) = 1 - V(t)$ et $F_2(V(t)) = V(t)$ sont déterminées à l'avance de la phase actuelle de ventilation sur la base des phases antérieures de ventilation.

Fig. 1

**Fig. 2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 102007062214 B3
- US 6588423 B1
- DE 10212497 A1
- US 2010083968 A
- US 2006272641 A
- US 2010252038 A
- US 2010180898 A

**Non-patent literature cited in the description**

- **MERLETTI R ; PARKER P.A.** Electromyography, Physiology, Engineering and Noninvasive Applications. IEEE Press, Wiley Interscience, 2004, 139